# EUROPÄISCHE PATENTANMELDUNG

(11) **EP 4 369 355 A1**
(43) Veröffentlichungstag der Anmeldung: **15.05.2024**
(21) Anmeldenummer: 23204546.8
(22) Anmeldetag: 19.10.2023
(51) Int. Cl.: G16H 50/20, A61M 16/00, G16H 20/40, G16H 40/63, G16H 40/67, H04L 67/00, G06N 20/00

(54) **DATENKOMMUNIKATION ZWISCHEN EINEM BEATMUNGSGERÄT UND MINDESTENS EINER DATENVERARBEITUNGSEINRICHTUNG**

(30) Priorität: 11.11.2022 DE 102022129950
(71) Anmelder: Löwenstein Medical Technology S.A., 2557 Luxembourg (LU)
(72) Erfinder: Schwaibold, Matthias, 76228 Karlsruhe (DE)
(74) Vertreter: Marx, Thomas

(57) **Zusammenfassung**

Ein Sendemodul (15) umfasst Mittel (19, 20) zum Ausführen der folgenden Schritte: Empfangen von Eingabedaten (11, 14), umfassend Sensordaten (11), die mindestens einen durch mindestens einen Sensor (10, 12) erfassten und für eine Beatmung eines Patienten durch ein Beatmungsgerät (2) relevanten Parameter definieren, und/oder umfassend Benutzerdaten (14), die durch einen Benutzer über eine Benutzerschnittstelle (13) eingegebene Informationen bezüglich eines durch ein Beatmungsgerät (2) beatmeten oder zu beatmenden Patienten codieren; Auswerten der Eingabedaten (11, 14), um ein Ereignis unter mehreren möglichen Ereignissen zu erkennen, wobei jedes mögliche Ereignis für eine Beatmung des Patienten durch das Beatmungsgerät (2) relevant ist; Senden einer Nachricht (16) bezüglich des erkannten Ereignisses an ein Empfangsmodul (18) über eine Datenkommunikationsverbindung (3), die das Beatmungsgerät (2) mit mindestens einer außerhalb des Beatmungsgeräts (2) befindlichen Datenverarbeitungseinrichtung (4, 5, 6) zur Datenkommunikation verbindet.

## Beschreibung

### Technisches Gebiet

Die Erfindung betrifft ein Sendemodul zum Senden einer Nachricht über eine Datenkommunikationsverbindung zwischen einem Beatmungsgerät und mindestens einer außerhalb des Beatmungsgeräts befindlichen Datenverarbeitungseinrichtung. Des Weiteren betrifft die Erfindung ein Empfangsmodul zum Empfangen einer Nachricht über eine solche Datenkommunikationsverbindung, ein entsprechendes Sende- bzw. Empfangsverfahren sowie ein Sende- und Empfangssystem, ein Computerprogramm und ein computerlesbares Medium zum Ausführen des Verfahrens. Zudem betrifft die Erfindung ein Beatmungsgerät und ein Beatmungssystem.

### Stand der Technik

Moderne Beatmungsgeräte wie Heimbeatmungsgeräte, Schlafatemtherapiegeräte, Intensivbeatmungsgeräte oder Anästhesiegeräte können mit einer zentralen Datenverarbeitungseinrichtung, beispielsweise einem (Cloud-)Server, vernetzt sein. Dies ermöglicht eine zentrale Überwachung der von einem Beatmungsgerät bereitgestellten Sensordaten. Mit dem Server können zusätzlich weitere Clients vernetzt sein, die Anfragen an den Server senden und/oder vom Server empfangen können und/oder über den Server mit dem Beatmungsgerät kommunizieren können, etwa um eine Fernwartung durchzuführen oder Beatmungsparameter aus der Distanz einzustellen. Dabei können je nach Anzahl der Netzwerkteilnehmer und je nach Typ der übertragenen Daten erhebliche Datenmengen anfallen, die das Datenkommunikationsnetzwerk stark belasten können oder sehr leistungsfähige und entsprechend teure Datenkommunikationsverbindungen erfordern.

### Offenbarung der Erfindung

Es ist daher Aufgabe der Erfindung, die Übertragung von Daten zwischen einem Beatmungsgerät und einem oder mehreren Datenverarbeitungseinrichtungen in einem Datenkommunikationsnetzwerk zu verbessern.

Diese Aufgabe wird durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein Sendemodul. Das Sendemodul umfasst Mittel zum Ausführen zumindest der folgenden Schritte: Empfangen von Eingabedaten, die Sensordaten und/oder Benutzerdaten umfassen, wobei die Sensordaten mindestens einen durch mindestens einen Sensor erfassten und für eine Beatmung eines Patienten durch ein Beatmungsgerät relevanten Parameter definieren und die Benutzerdaten durch einen Benutzer über eine Benutzerschnittstelle eingegebene Informationen bezüglich eines durch ein Beatmungsgerät beatmeten oder zu beatmenden Patienten codieren; Auswerten der Eingabedaten, um ein Ereignis unter mehreren möglichen Ereignissen zu erkennen, wobei jedes mögliche Ereignis für eine Beatmung des Patienten durch das Beatmungsgerät relevant ist; Senden einer Nachricht bezüglich des erkannten Ereignisses an ein Empfangsmodul über eine Datenkommunikationsverbindung, die das Beatmungsgerät mit mindestens einer außerhalb des Beatmungsgeräts befindlichen Datenverarbeitungseinrichtung zur Datenkommunikation verbindet.

Anders ausgedrückt ermöglicht der hier vorgestellte Ansatz eine ereignisgesteuerte Datenkommunikation zwischen dem Beatmungsgerät und einer Datenverarbeitungseinrichtung (oder mehreren Datenverarbeitungseinrichtungen), indem Daten in Form von Nachrichten nur dann übertragen werden, wenn ein bestimmtes, für die Beatmung relevantes Ereignis erkannt wird. Dies hat den Effekt, dass die Menge der übertragenen Daten im Vergleich zu Ausführungen, bei denen Nachrichten in bestimmten Zeitintervallen unabhängig vom Nachrichteninhalt übertragen werden, deutlich verringert wird, was die Effizienz der Datenkommunikation verbessert und Überlastungen des Datenkommunikationsnetzwerks verhindert. Ein weiterer Vorteil ist, dass insgesamt weniger Kosten bei der Datenkommunikation anfallen, da kostengünstigere Datentarife und/oder weniger leistungsfähige Hardwarekomponenten ohne maßgebliche Funktionseinbußen oder Informationsverluste verwendet werden können. Insbesondere in sehr großen Datenkommunikationsnetzwerken, beispielsweise innerhalb einer großen Klinik oder wenn Hunderte oder sogar Tausende von (Heim-)Beatmungsgeräten von verschiedenen Standorten aus mit einem zentralen Server zu Überwachungs- und/oder Wartungszwecken kommunizieren, kann die Verringerung der zu übertragenden (Nutz-)Daten, wie sie der hier vorgestellte Ansatz ermöglicht, einen erheblichen Einfluss auf die Kosten haben.

Im Folgenden werden bestimmte Begriffe näher erläutert.

Unter "Modul" wie in "Sendemodul" oder "Empfangsmodul" kann ein Soft- und/oder Hardwaremodul verstanden werden. Ein solches Modul kann eine Komponente des Beatmungsgeräts und/oder der Datenverarbeitungseinrichtung sein. Beispielsweise kann das Sende- bzw. Empfangsmodul als Softwaremodul einer speziellen Steuerungs- und/oder Überwachungssoftware und/oder einer speziellen App im Beatmungsgerät bzw. in der Datenverarbeitungseinrichtung ausgeführt werden. Alternativ kann das Sende- bzw. Empfangsmodul ein Hardwaremodul mit einem Prozessor sein, wobei der Prozessor konfiguriert sein kann, um ein bestimmtes Computerprogramm (siehe weiter unten) auszuführen.

Unter "Beatmungsgerät" kann beispielsweise ein Heimbeatmungsgerät, ein Schlafatemtherapiegerät, ein Intensivbeatmungsgerät, ein Anästhesiegerät oder eine Kombination aus mindestens zwei dieser Beispiele verstanden werden. Das Beatmungsgerät kann zur invasiven und/oder nicht invasiven Beatmung des Patienten geeignet sein. Das Beatmungsgerät kann vom Patienten tragbar oder stationär sein.

Unter "Datenverarbeitungseinrichtung" kann allgemein eine einen Computer umfassende Einrichtung verstanden werden. Demnach kann die Datenverarbeitungseinrichtung einen Prozessor, einen Speicher und Datenkommunikationsschnittstellen zur drahtlosen und/oder drahtgebundenen Datenkommunikation mit Peripheriegeräten, d. h. dem Beatmungsgerät und/oder anderen Datenverarbeitungseinrichtungen, umfassen. Die Datenverarbeitungseinrichtung kann beispielsweise ein (Cloud-)Server, ein PC, ein Laptop, ein Tablet, ein Smartphone, eine Smartwatch oder eine Kombination aus mindestens zwei dieser Beispiele sein. Die Datenverarbeitungseinrichtung kann aber auch ein weiteres Beatmungsgerät sein.

Unter "Sensor" kann eine Komponente des Beatmungsgeräts selbst oder ein vom Beatmungsgerät getrennter, beispielsweise vom Patienten getragener Sensor verstanden werden. Die Sensordaten können insbesondere unter Verwendung verschiedener Typen von Sensoren bereitgestellt worden sein. Ein solcher Sensor kann beispielsweise als Drucksensor, Volumenstromsensor, Feuchtigkeitssensor, Pulsoximeter oder Thermometer ausgeführt sein. Ein Atemgasvolumenstrom (auch Fluss genannt) kann beispielsweise durch Messung eines Differenzdrucks, durch thermische Anemometrie, mit einem Ultraschallverfahren oder basierend auf einer Kombination von mindestens zwei dieser Beispiele bestimmt werden. Für die Messung einer Atemgaszusammensetzung können beispielsweise infrarotoptische, spektroskopische, elektromagnetische oder elektrochemische Verfahren oder mehrere dieser Verfahren in Kombination eingesetzt werden.

Unter "Benutzer" kann der Patient selbst oder eine andere Person als der Patient, beispielsweise ein Bediener des Beatmungsgeräts oder ein Arzt oder ein Betreuer des Patienten, verstanden werden.

Unter "Benutzerdaten" können allgemein Daten mit Bezug auf einen Gesundheitszustand des Patienten verstanden werden. Konkret können die Benutzerdaten beispielsweise Aufschluss über einen Therapieverlauf, eine Compliance, eine Diagnose, eine Anamnese und/oder eine Medikation des Patienten geben. Anders ausgedrückt können die Benutzerdaten Ergebnisse einer oder mehrerer medizinischer Untersuchungen des Patienten und/oder Antworten des Patienten (oder allgemein eines Bedieners des Beatmungsgeräts) auf bestimmte für die Beatmung relevante Fragen codieren. Solche Fragen können beispielsweise über die Benutzerschnittstelle (z. B. in einer entsprechenden App) angezeigt und/oder beantwortet worden sein.

Zusätzlich können die Eingabedaten Logdaten oder Fehlermeldungen des Beatmungsgeräts umfassen.

Unter "Benutzerschnittstelle" kann allgemein eine Mensch-Maschine-Schnittstelle verstanden werden. Beispielsweise kann die Benutzerschnittstelle eine auf einem (Touch-)Display anzeigte Benutzeroberfläche einer speziellen App, die auf dem Beatmungsgerät und/oder der (damit vernetzten) Datenverarbeitungseinrichtung läuft, und/oder separate Bedienelemente wie eine Tastatur oder eine Maus umfassen. Die Benutzerschnittstelle kann eine Hard- und/oder Softwarekomponente des Beatmungsgeräts und/oder der mindestens einen Datenverarbeitungseinrichtung sein. Die Benutzerschnittstelle kann aber auch eine Hard- und/oder Softwarekomponente eines mit dem Beatmungsgerät und/oder der mindestens einen Datenverarbeitungseinrichtung zur Datenkommunikation verbundenen (zusätzlichen) Benutzergeräts sein. Ein solches Benutzergerät kann beispielsweise ein PC, ein Laptop, ein Tablet oder ein Smartphone des Benutzers sein. In diesem Fall fungiert also das Benutzergerät als die Benutzerschnittstelle des Beatmungsgeräts und/oder der mindestens einen Datenverarbeitungseinrichtung.

Unter "Ereignis" kann ein medizinisches und/oder technisches Ereignis verstanden werden. Beispielsweise kann das erkannte Ereignis eine medizinische und/oder technische Komplikation sein, die das Risiko für eine signifikante Verschlechterung des Gesundheitszustands des Patienten, insbesondere seiner Atemfähigkeit, erhöhen kann. Als Ereignis kann aber auch eine signifikante Verbesserung des Gesundheitszustands des Patienten, insbesondere seiner Atemfähigkeit, erkannt werden (Beispiele für mögliche Ereignisse werden weiter unten näher beschrieben).

Die Datenkommunikationsverbindung kann eine drahtlose und/oder drahtgebundene Datenkommunikation in einem LAN, WAN und/oder GAN ermöglichen. Beispielsweise kann die Datenkommunikationsverbindung mindestens eine der folgenden Verbindungen sein: eine Internetverbindung, eine WLAN-Verbindung, eine Mobilfunkverbindung, eine Bluetooth-Verbindung.

Die Datenkommunikationsverbindung kann das Beatmungsgerät und die Datenverarbeitungseinrichtung(en) indirekt, d. h. mittels mindestens einer weiteren Datenverarbeitungseinrichtung, und/oder direkt, d. h. ohne Umweg über eine oder mehrere weitere Datenverarbeitungseinrichtungen, miteinander verbinden.

Beispielsweise kann die Datenkommunikationsverbindung das Beatmungsgerät über einen Server, also indirekt, mit einem (mobilen) Endgerät als der Datenverarbeitungseinrichtung (z. B. einem PC, einem Laptop, einem Tablet oder einem Smartphone) verbinden. Möglich ist aber auch eine direkte Datenkommunikationsverbindung zwischen dem Beatmungsgerät und dem Endgerät.

Eine weitere Möglichkeit ist, dass die Datenkommunikationsverbindung das Beatmungsgerät über ein (mobiles) Endgerät (z. B. einem PC, einem Laptop, einem Tablet oder einem Smartphone), also indirekt, mit einem Server als der Datenverarbeitungseinrichtung verbindet. Beispielsweise kann das Beatmungsgerät, bevorzugt über Bluetooth und/oder WLAN, mit dem Endgerät gekoppelt sein, um Daten mit dem Endgerät auszutauschen, wobei das Endgerät, bevorzugt über das Internet, mit dem Server gekoppelt sein kann, um Daten mit dem Server auszutauschen. Möglich ist aber auch eine direkte Datenkommunikationsverbindung zwischen dem Beatmungsgerät und dem Server.

Die Eingabedaten können zumindest teilweise über die Datenkommunikationsverbindung im Sendemodul empfangen werden. Beispielsweise kann zumindest ein Teil der Eingabedaten, d. h. zumindest ein Teil der Sensor- und/oder der Benutzerdaten, in der Datenverarbeitungseinrichtung vom Beatmungsgerät empfangen werden (oder umgekehrt).

Ein zweiter Aspekt der Erfindung betrifft ein Empfangsmodul. Das Empfangsmodul umfasst Mittel zum Ausführen zumindest der folgenden Schritte: Empfangen einer Nachricht bezüglich eines für eine Beatmung eines Patienten durch ein Beatmungsgerät relevanten Ereignisses von einem Sendemodul, beispielsweise dem vor- und nachstehenden beschriebenen Sendemodul, über eine Datenkommunikationsverbindung, die das Beatmungsgerät mit mindestens einer außerhalb des Beatmungsgeräts befindlichen Datenverarbeitungseinrichtung zur Datenkommunikation verbindet; Erzeugen eines ersten Steuerbefehls zum Ansteuern einer Benutzerschnittstelle, sodass eine für einen Benutzer relevante Information aus der Nachricht über die Benutzerschnittstelle wiedergegeben wird, und/oder Erzeugen eines zweiten Steuerbefehls zum Ansteuern eines Aktors des Beatmungsgeräts unter Verwendung der Nachricht, wobei der Aktor ausgebildet ist, um einen Atemgasdruck und/oder einen Atemgasvolumenstrom zum Beatmen des Patienten bereitzustellen.

Anders ausgedrückt kann die Nachricht zur Kommunikation mit dem Benutzer, also dem Patienten oder allgemein dem Bediener des Beatmungsgeräts, und/oder zur Steuerung einer oder mehrerer Beatmungsfunktionen des Beatmungsgeräts ausgewertet werden.

Wie weiter oben erwähnt, kann das Empfangsmodul eine Hard- und/oder Softwarekomponente des Beatmungsgeräts und/oder der Datenverarbeitungseinrichtung sein. Denkbar ist beispielsweise, dass die Datenverarbeitungseinrichtung den ersten und/oder zweiten Steuerbefehl über die Datenkommunikationsverbindung ans Beatmungsgerät sendet, wo der jeweilige Steuerbefehl entsprechend verarbeitet und umgesetzt wird. Der erste und/oder zweite Steuerbefehl kann aber auch im Beatmungsgerät erzeugt werden.

Die für den Benutzer relevante Information kann bei der Wiedergabe über die Benutzerschnittstelle beispielsweise als Text, Bild und/oder Video auf einem Display angezeigt und/oder als Sprache und/oder Töne über einen Lautsprecher ausgegeben werden.

Es ist möglich, dass der zweite Steuerbefehl entsprechend einer aktuellen Einstellung mindestens eines Steuerparameters erzeugt wird. Die Einstellung kann beispielsweise unter Verwendung von in der Nachricht enthaltenen Referenzwerten geändert werden, um die Beatmungsfunktion zu verbessern.

Ein dritter Aspekt der Erfindung betrifft ein Sende- und Empfangssystem, das ein Sendemodul wie vor- und nachstehend beschrieben und ein Empfangsmodul wie vor- und nachstehend beschrieben umfasst. Die Komponenten des Sende- und Empfangssystems können Komponenten verschiedener (miteinander vernetzter) Geräte und/oder ein und desselben Geräts sein. Beispielsweise kann die Datenverarbeitungseinrichtung oder können die Datenverarbeitungseinrichtungen jeweils das Empfangsmodul umfassen, während das Beatmungsgerät das Sendemodul umfassen kann. Möglich ist aber auch der umgekehrte Fall. Alternativ können sowohl das Beatmungsgerät als auch die Datenverarbeitungseinrichtung(en) jeweils eine Kombination aus einem Sende- und einem Empfangsmodul (d. h. das Sende- und Empfangssystem) umfassen.

Ein vierter Aspekt der Erfindung betrifft ein Beatmungsgerät. Das Beatmungsgerät umfasst: einen Sensor zum Erfassen von mindestens einem für eine Beatmung eines Patienten durch das Beatmungsgerät relevanten Parameter; eine Datenkommunikationsschnittstelle zum Verbinden des Beatmungsgeräts mit mindestens einer außerhalb des Beatmungsgeräts befindlichen Datenverarbeitungseinrichtung zur Datenkommunikation; eine Benutzerschnittstelle zum Eingeben von Informationen durch einen Benutzer und/oder zum Wiedergeben von für einen Benutzer relevanten Informationen; einen Aktor zum Bereitstellen eines Atemgasdrucks und/oder eines Atemgasvolumenstroms zum Beatmen des Patienten; ein Sendemodul wie vor- und nachstehend beschrieben und/oder ein Empfangsmodul wie vor- und nachstehend beschrieben.

Der Aktor kann beispielsweise ein Ventilator mit Balg- und/oder Kolbenantrieb und/oder in Form eines Radialverdichters sein.

Der Aktor kann zusätzlich ausbildet sein, um eine Atemgaszusammensetzung, beispielsweise eine Sauerstoff-, Kohlendioxid- und/oder Anästhesiegaskonzentration im Atemgas, zu steuern.

Ein fünfter Aspekt der Erfindung betrifft ein Beatmungssystem. Das Beatmungssystem umfasst ein Beatmungsgerät wie vor- und nachstehend beschrieben sowie eine außerhalb des Beatmungsgeräts befindliche Datenverarbeitungseinrichtung (oder mehrere solcher Datenverarbeitungseinrichtungen). Die Datenverarbeitungseinrichtung umfasst eine Datenkommunikationsschnittstelle zum Verbinden der mindestens einen Datenverarbeitungseinrichtung mit dem Beatmungsgerät zur Datenkommunikation, eine Benutzerschnittstelle zum Eingeben von Informationen durch einen Benutzer und/oder zum Wiedergeben von für einen Benutzer relevanten Informationen und mindestens eines der vor- und nachstehend beschriebenen Module zum Senden bzw. Empfangen von Nachrichten über die Datenkommunikationsschnittstelle.

Das Beatmungsgerät und die Datenverarbeitungseinrichtung(en) können geografisch gesehen mehrere Meter, mehrere Kilometer oder sogar mehrere Hundert oder mehrere Tausend Meter oder Kilometer voneinander entfernt sein.

Ein sechster Aspekt der Erfindung betrifft ein Computerprogramm.

Das Computerprogramm umfasst einen ersten Satz von Befehlen, die ein Sendemodul bei der Ausführung des Computerprogramms durch das Sendemodul veranlassen, zumindest die folgenden Schritte auszuführen: Empfangen von Eingabedaten, die Sensordaten und/oder Benutzerdaten umfassen, wobei die Sensordaten mindestens einen durch mindestens einen Sensor erfassten und für eine Beatmung eines Patienten durch ein Beatmungsgerät relevanten Parameter definieren und die Benutzerdaten durch einen Benutzer über eine Benutzerschnittstelle eingegebene Informationen bezüglich eines durch ein Beatmungsgerät beatmeten oder zu beatmenden Patienten codieren; Auswerten der Eingabedaten, um ein Ereignis unter mehreren möglichen Ereignissen zu erkennen, wobei jedes mögliche Ereignis für eine Beatmung des Patienten durch das Beatmungsgerät relevant ist; Senden einer Nachricht bezüglich des erkannten Ereignisses an ein Empfangsmodul, beispielsweise das vor- und nachstehende beschriebene Empfangsmodul, über eine Datenkommunikationsverbindung, die das Beatmungsgerät mit mindestens einer außerhalb des Beatmungsgeräts befindlichen Datenverarbeitungseinrichtung zur Datenkommunikation verbindet.

Zusätzlich oder alternativ zum ersten Satz umfasst das Computerprogramm einen zweiten Satz von Befehlen, die ein Empfangsmodul bei der Ausführung des Computerprogramms durch das Empfangsmodul veranlassen, zumindest die folgenden Schritte auszuführen: Empfangen einer Nachricht bezüglich eines für eine Beatmung eines Patienten durch ein Beatmungsgerät relevanten Ereignisses von einem Sendemodul, beispielsweise dem vor- und nachstehenden beschriebenen Sendemodul, über eine Datenkommunikationsverbindung, die das Beatmungsgerät mit mindestens einer außerhalb des Beatmungsgeräts befindlichen Datenverarbeitungseinrichtung zur Datenkommunikation verbindet; Erzeugen eines ersten Steuerbefehls zum Ansteuern einer Benutzerschnittstelle, sodass eine für einen Benutzer relevante Information aus der Nachricht über die Benutzerschnittstelle wiedergegeben wird, und/oder Erzeugen eines zweiten Steuerbefehls zum Ansteuern eines Aktors des Beatmungsgeräts unter Verwendung der Nachricht, wobei der Aktor ausgebildet ist, um einen Atemgasdruck und/oder einen Atemgasvolumenstrom zum Beatmen des Patienten bereitzustellen.

Unter "Computerprogramm" kann beispielsweise eine spezielle Steuerungs- und/oder Überwachungssoftware und/oder eine spezielle App, die auf dem Beatmungsgerät und/oder der Datenverarbeitungseinrichtung (oder den Datenverarbeitungseinrichtungen) ausgeführt werden kann, verstanden werden.

Ein siebter Aspekt der Erfindung betrifft ein computerlesbares Medium, auf dem das Computerprogramm gespeichert ist. Das computerlesbare Medium kann ein flüchtiger oder nicht flüchtiger Datenspeicher sein. Beispielsweise kann das computerlesbare Medium eine Festplatte, ein USB-Speichergerät (*universal serial bus*)*,* ein RAM (*random-access memory*)*,* ein ROM (*read-only memory*)*,* ein EPROM (*erasable programmable read-only memory*)*,* ein EEPROM (*electrically erasable programmable read-only memory*)*,* ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale des Sendemoduls, des Empfangsmoduls und/oder des Sende- und Empfangssystems wie vor- und nachstehend beschrieben auch Merkmale des Computerprogramms und/oder des computerlesbaren Mediums sein können (und umgekehrt).

Ein achter Aspekt der Erfindung betrifft ein Verfahren zum Betreiben eines Sendemoduls und/oder eines Empfangsmoduls.

Das Verfahren umfasst zumindest die folgenden Schritte zum Betreiben des Sendemoduls: Empfangen von Eingabedaten, die Sensordaten und/oder Benutzerdaten umfassen, wobei die Sensordaten mindestens einen durch mindestens einen Sensor erfassten und für eine Beatmung eines Patienten durch ein Beatmungsgerät relevanten Parameter definieren und die Benutzerdaten durch einen Benutzer über eine Benutzerschnittstelle eingegebene Informationen bezüglich eines durch ein Beatmungsgerät beatmeten oder zu beatmenden Patienten codieren; Auswerten der Eingabedaten, um ein Ereignis unter mehreren möglichen Ereignissen zu erkennen, wobei jedes mögliche Ereignis für eine Beatmung des Patienten durch das Beatmungsgerät relevant ist; Senden einer Nachricht bezüglich des erkannten Ereignisses an ein Empfangsmodul, beispielsweise das vor- und nachstehende beschriebene Empfangsmodul, über eine Datenkommunikationsverbindung, die das Beatmungsgerät mit mindestens einer außerhalb des Beatmungsgeräts befindlichen Datenverarbeitungseinrichtung zur Datenkommunikation verbindet.

Zusätzlich oder alternativ umfasst das Verfahren zumindest die folgenden Schritte zum Betreiben des Empfangsmoduls: Empfangen einer Nachricht bezüglich eines für eine Beatmung eines Patienten durch ein Beatmungsgerät relevanten Ereignisses von einem Sendemodul, beispielsweise dem vor- und nachstehenden beschriebenen Sendemodul, über eine Datenkommunikationsverbindung, die das Beatmungsgerät mit mindestens einer außerhalb des Beatmungsgeräts befindlichen Datenverarbeitungseinrichtung zur Datenkommunikation verbindet; Erzeugen eines ersten Steuerbefehls zum Ansteuern einer Benutzerschnittstelle, sodass eine für einen Benutzer relevante Information aus der Nachricht über die Benutzerschnittstelle wiedergegeben wird, und/oder Erzeugen eines zweiten Steuerbefehls zum Ansteuern eines Aktors des Beatmungsgeräts unter Verwendung der Nachricht, wobei der Aktor ausgebildet ist, um einen Atemgasdruck und/oder einen Atemgasvolumenstrom zum Beatmen des Patienten bereitzustellen.

Das Verfahren kann computerimplementiert sein.

Es wird darauf hingewiesen, dass Merkmale des Sendemoduls, des Empfangsmoduls, des Sende- und Empfangssystems, des Computerprogramms und/oder des computerlesbaren Mediums wie vor- und nachstehend beschrieben auch Merkmale des Verfahrens sein können (und umgekehrt).

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann ein erster Satz der Eingabedaten empfangen werden. In diesem Fall können die Eingabedaten aus dem ersten Satz ausgewertet werden, um das Ereignis zu erkennen. Wird das Ereignis erkannt, so kann das Auswerten der Eingabedaten zusätzlich die folgenden Plausibilisierungsschritte umfassen: Anfordern mindestens eines vom ersten Satz abweichenden zweiten Satzes der Eingabedaten und, wenn der zweite Satz empfangen wird, Prüfen anhand der Eingabedaten aus dem zweiten Satz, ob das erkannte Ereignis plausibel ist, wobei ein Prüfergebnis erzeugt wird. Die Nachricht kann dann abhängig vom Prüfergebnis gesendet werden.

Im einfachsten Fall kann das Prüfergebnis eine binäre Information wie "plausibel" oder "nicht plausibel" sein. Das Prüfergebnis kann aber auch eine Wahrscheinlichkeit bezüglich der Plausibilität des erkannten Ereignisses anzeigen. Die Wahrscheinlichkeit kann dann beispielsweise mit einem Schwellenwert verglichen werden, um zu bestimmen, ob das erkannte Ereignis plausibel ist oder nicht.

Zweckmäßigerweise kann das Senden der Nachricht unterbunden werden, wenn das Prüfergebnis anzeigt, dass das erkannte Ereignis nicht plausibel ist. Die Nachricht wird also nur dann gesendet, wenn es sehr wahrscheinlich ist, dass das erkannte Ereignis ein reales Ereignis ist. Dies verbessert zum einen die Effizienz der Datenübertragung gegenüber Ausführungen ohne eine derartige Plausibilisierung, da insgesamt weniger Nachrichten, nämlich nur Nachrichten, die sich auf plausible Ereignisse beziehen, gesendet werden. Zum anderen verbessert dies die Erkennungsgenauigkeit.

Gemäß einer Ausführungsform kann der zweite Satz andere Parameter als der erste Satz definieren. Anders ausgedrückt kann zur Plausibilisierung des erkannten Ereignisses auf einen anderen Typ oder andere Typen von Sensordaten als zum (erstmaligen) Erkennen des Ereignisses zurückgegriffen werden, beispielsweise auf Sensordaten, die ein außerhalb des Beatmungsgeräts befindlicher und/oder vom Patienten getragener zusätzlicher Sensor bereitstellt. Wird zur Plausibilisierung auf den gleichen Typ oder die gleichen Typen von Sensordaten wie zur Erkennung zurückgegriffen, so kann es sein, dass die Sensordaten in beiden Fällen ähnlich fehlerhaft sind, etwa aufgrund eines Defekts oder einer Fehlfunktion des betreffenden Sensors. Mithilfe dieser Ausführungsform können solche Fehler vermieden werden.

Gemäß einer Ausführungsform kann das Auswerten der Eingabedaten die folgenden Schritte umfassen: Umwandeln zumindest eines Teils der Eingabedaten als Klassifikationsdaten in Klassenwerte durch einen Klassifikator, wobei jeder Klassenwert eine Wahrscheinlichkeit und/oder Tendenz für eine von mehreren Ereignisklassen anzeigt, wobei jede Ereignisklasse einem der möglichen Ereignisse entspricht; Erkennen des Ereignisses unter Verwendung der Klassenwerte.

Unter "Klassenwert" kann beispielsweise ein Prozentwert zwischen 0 und 1, ein boolescher Wert wie "0" oder "1" oder ein Tendenzwert aus einem kontinuierlichen Wertebereich verstanden werden.

Der Klassifikator kann beispielsweise ein (naiver) Bayes-Klassifikator, ein *k*-Nächste-Nachbarn-Algorithmus, eine Support-Vector-Maschine, ein Clusterverfahren, ein künstliches neuronales Netz (z. B. ein mehrlagiges Perzeptron, ein *convolutional neural network,* ein *recurrent neural network,* ein *long short-term memory*) oder eine Kombination aus mindestens zwei dieser Beispiele sein.

Das erkannte Ereignis kann beispielsweise der Ereignisklasse mit dem größten oder kleinsten Klassenwert entsprechen.

Beispielsweise kann zum Auswerten der Eingabedaten aus dem ersten Satz (siehe weiter oben) ein anderer, beispielsweise anders trainierter und/oder anders strukturierter Klassifikator als zum Auswerten der Eingabedaten aus dem zweiten Satz verwendet werden.

Gemäß einer Ausführungsform kann der Klassifikator ein künstliches neuronales Netz (siehe weiter oben) umfassen, das mit beispielhaften Klassifikationsdaten trainiert wurde, um die Klassifikationsdaten in die Klassenwerte umzuwandeln. Bei den beispielhaften Klassifikationsdaten kann es sich um Trainingsdaten in Form aufgezeichneter und entsprechend vorverarbeiteter Sensor- und/oder Benutzerdaten handeln. Das Netz kann in einem überwachten Lernverfahren trainiert worden sein, bei dem Ausgabewerte des Netzes mit bekannten Zielwerten (auch Labels genannt) verglichen werden, eine Diskrepanz zwischen den Ausgabe- und den Zielwerten mit einer Verlustfunktion bestimmt wird und die Verlustfunktion durch iteratives Anpassen der Gewichte des Netzes minimiert wird, beispielsweise in einem (stochastischen) Gradientenverfahren. Möglich ist aber auch ein unüberwachtes Lernverfahren. Ein solcher Klassifikator ermöglicht eine sehr genaue Erkennung. Darüber hinaus lässt sich ein solcher Klassifikator durch Trainieren mit neuen Trainingsdaten mit geringem Aufwand aktualisieren, etwa um die Erkennung neuer Ereignisse zu ermöglichen.

Gemäß einer Ausführungsform kann eine Abweichung des mindestens einen Parameters von mindestens einem Sollwert bestimmt werden. Dabei kann das Ereignis abhängig von der Abweichung erkannt werden. Anders ausgedrückt kann ein Schwellenwertvergleich oder können mehrere Schwellenwertvergleiche zum Auswählen eines bestimmten Ereignisses aus der Menge der möglichen Ereignisse durchgeführt werden. Diese Schwellen- oder Sollwerte können beispielsweise Erfahrungswerte sein und/oder in Versuchen ermittelt worden sein.

Gemäß einer Ausführungsform kann jedem möglichen Ereignis eine Priorität zugeordnet sein und die Nachricht entsprechend der Priorität des erkannten Ereignisses gesendet werden. Dabei können sich die Prioritäten unterschiedlicher Ereignisse voneinander unterscheiden. Dies ermöglicht es, wichtige Ereignisse gegenüber weniger wichtigen Ereignissen bevorzugt zu behandeln. Anders ausgedrückt können Nachrichten, die sich auf unterschiedliche erkannte Ereignisse beziehen, mit unterschiedlicher Priorität gesendet werden. Beispielsweise kann eine Nachricht nur dann gesendet werden, wenn die Priorität des durch die Nachricht angezeigten Ereignisses größer als ein bestimmter Schwellenwert ist. Somit kann eine Vorauswahl an zu sendenden Nachrichten getroffen werden. Dies kann die Effizienz der Datenkommunikation weiter verbessern. Zudem kann dadurch das Risiko von Datenverlusten gegenüber Ausführungen, bei denen alle Nachrichten mit der gleichen Priorität versendet werden, verringert werden.

Gemäß einer Ausführungsform kann ein Wert für mindestens einen Datenparameter abhängig vom erkannten Ereignis bestimmt werden, wobei der mindestens eine Datenparameter die Menge an Daten, die beim Senden der Nachricht gesendet werden, definiert.

Anders ausgedrückt kann durch Einstellen des Datenparameters oder der Datenparameter die Belastung der Datenkommunikationsverbindung beim Senden der Nachricht gezielt beeinflusst werden. Beim Senden der (gleichen) Nachricht können also mehr oder weniger Daten versendet werden, je nachdem, welche Einstellung für den oder die Datenparameter gewählt wurde. Der Datenverbrauch kann somit sehr genau abhängig vom Nachrichteninhalt gesteuert werden.

Unter "Datenparameter" kann beispielsweise ein Datendurchsatz beim Senden der Nachricht, eine maximale Größe der Nachricht, ein Dateiformat der Nachricht, ein Parameter einer Komprimierungsmethode zum Komprimieren der Nachricht oder ein Parameter einer Verschlüsselungsmethode zum Verschlüsseln der Nachricht verstanden werden.

Der Wert für den oder die Datenparameter kann zusätzlich unter Berücksichtigung einer aktuellen Auslastung der Datenkommunikationsverbindung und/oder einer dem erkannten Ereignis zugeordneten Priorität (siehe weiter oben) bestimmt werden. So kann beispielsweise beim Senden einer Nachricht bezüglich eines Ereignisses mit höherer Priorität eine größere Datenmenge übertragen werden, als wenn sich die Nachricht auf ein Ereignis mit niedrigerer Priorität bezieht. Beispielsweise kann die Nachricht dann höher aufgelöste Sensordaten als bei niedrigerer Priorität umfassen. Möglich ist aber auch der umgekehrte Fall, etwa bei starker Auslastung der Datenkommunikationsverbindung. Hier kann es unter Umständen sinnvoll sein, wichtige Nachrichten stärker als weniger wichtige Nachrichten zu komprimieren, etwa um das Risiko von Datenverlusten zu verringern und/oder zu erreichen, dass die Nachrichten schneller beim jeweiligen Empfänger ankommen.

Auch kann es sinnvoll sein, Nachrichten mit vertraulichen Inhalten (die eine höhere Priorität haben können) verschlüsselt zu versenden, wohingegen Nachrichten mit anderen, nicht vertraulichen Inhalten (die eine niedrigere Priorität haben können) unverschlüsselt versendet werden können.

Wird die Nachricht in Datenpaketen gesendet, so ist es zweckmäßig, wenn beim Senden jedes Datenpakets geprüft wird, ob das Datenpaket erfolgreich empfangen wird. Jedes Datenpaket, das nicht erfolgreich empfangen wurde, kann dann mindestens ein weiteres Mal gesendet werden. Somit können Datenverluste beim Senden der Nachricht vermieden werden.

Beispielsweise kann ein Empfänger der Datenpakete konfiguriert sein, um für jedes Datenpaket, das der Empfänger erfolgreich empfängt, eine Empfangsbestätigung über die Datenkommunikationsverbindung zu senden. Wird die Empfangsbestätigung innerhalb einer bestimmten Wartezeit seit dem letzten Senden des Datenpakets empfangen, so kann das Datenpaket als erfolgreich empfangen markiert werden. Dann kann das nächste Datenpaket gesendet werden. Wird die Empfangsbestätigung hingegen nicht innerhalb der Wartezeit empfangen, so kann das Datenpaket erneut gesendet werden, beispielsweise so oft, bis die Empfangsbestätigung empfangen wird und/oder eine zulässige Anzahl an Sendeversuchen überschritten wird.

Kann das Datenpaket auch nach erneutem Senden nicht erfolgreich empfangen werden, so kann beispielsweise eine Nachricht an einen Benutzer generiert werden, die den Benutzer auf einen möglichen Datenverlust hinweist und/oder Informationen für ein Debugging umfasst.

Gemäß einer Ausführungsform können die möglichen Ereignisse mindestens eines der folgenden Ereignisse umfassen:
- eine Abweichung einer Beatmungsfrequenz, mit der der Patient beatmet wird, von einer Atemfrequenz, mit der der Patient atmet, liegt außerhalb eines Sollbereichs;
- ein inspiratorischer und/oder exspiratorischer Druck liegt außerhalb eines Sollbereichs;
- eine Geschwindigkeit, mit der sich ein Druck beim Übergang zwischen Inspiration und Exspiration ändert, liegt außerhalb eines Sollbereichs;
- ein Druckverlust bei der Beatmung liegt außerhalb eines Sollbereichs;
- der Patient hat Atemnot bei der Beatmung und/oder als Reaktion auf die Beendigung der Beatmung;
- das Beatmungsgerät erschwert das Aus- und/oder Einatmen;
- der Patient hat Schlafprobleme (z. B. bei der Beatmung);
- der Patient hat Kopfschmerzen (z. B. bei der Beatmung);
- der Patient hat zu trockene Atemwege (z. B. bei der Beatmung);
- der Patient ist erkältet;
- eine bestehende Erkrankung des Patienten hat sich signifikant verschlechtert oder verbessert;
- der Patient hat Schwierigkeiten bei der Bedienung des Beatmungsgeräts;
- die Compliance des Patienten ist unzureichend.

Gemäß einer Ausführungsform kann die Nachricht mindestens eine der folgenden Informationen umfassen:
- zumindest einen Teil der ausgewerteten Eingabedaten;
- eine Beschreibung des erkannten Ereignisses (eine solche Beschreibung kann beispielsweise auch eine Aufforderung an den Patienten beinhalten, einen bestimmten zusätzlichen Sensor zur Erfassung relevanter Sensordaten anzulegen und/oder zu aktivieren);
- eine Aufforderung an einen Benutzer, Informationen bezüglich des erkannten Ereignisses über eine Benutzerschnittstelle einzugeben (eine solche Aufforderung kann beispielsweise eine Liste von an den Patienten gerichteten Fragen beinhalten);
- eine Aufforderung an den Patienten, Kontakt mit einer Kontaktperson aufzunehmen, insbesondere über einen Chat- und/oder Videokommunikationsdienst;
- eine Anleitung, wie eine bestimmte Komplikation bei der Beatmung beseitigt und/oder verhindert werden kann;
- eine Ermunterung, die den Patienten zu einer besseren Compliance motivieren soll;
- einen an das erkannte Ereignis angepassten (Referenz-)Wert für mindestens einen Steuerparameter zum Steuern eines Aktors des Beatmungsgeräts;
- eine Patientenkennung zur eindeutigen Identifizierung des Patienten;
- eine Gerätekennung zur eindeutigen Identifizierung des Beatmungsgeräts.

Gemäß einer Ausführungsform können die Sensordaten mindestens einen der folgenden Parameter bezüglich des Patienten definieren: einen inspiratorischen und/oder exspiratorischen Druck, einen inspiratorischen und/oder exspiratorischen Volumenstrom, ein Atemgasvolumen pro Atemzug, eine Inspirations- und/oder Exspirationszeit, eine Beatmungsfrequenz, eine Atemfrequenz, eine Atemgaszusammensetzung, eine Atemanstrengung (auch Effort genannt), ein Atemgeräusch, eine Herzfrequenz, eine Blutsauerstoffsättigung, einen Blutdruck, einen Blutzuckerspiegel, eine Körpertemperatur, ein Körpergewicht, eine Körperbewegung.

Unter "Körperbewegung" kann beispielsweise eine Rumpf-, Arm-, Bein- oder Kopfbewegung oder eine Gehbewegung verstanden werden.

Gemäß einer Ausführungsform können die Sensordaten einen zeitlichen Verlauf des mindestens einen Parameters definieren, beispielsweise eine Atemdruck- und/oder Atemflusskurve. Anders ausgedrückt können die Sensordaten in unterschiedlichen Zeiträumen erzeugt worden sein. Das Ereignis kann also basierend auf einer Zeitreihe von Messwerten bezüglich des Parameters oder der Parameter erkannt werden, was die Erkennungsgenauigkeit gegenüber Ausführungen ohne Berücksichtigung des zeitlichen Verlaufs verbessert.

Gemäß einer Ausführungsform kann die Datenkommunikationsschnittstelle des Beatmungsgeräts konfiguriert sein, um das Beatmungsgerät zusätzlich mit mindestens einem außerhalb des Beatmungsgeräts befindlichen und/oder vom Patienten getragenen zusätzlichen Sensor zum Erfassen von mindestens einem für die Beatmung des Patienten relevanten Parameter zur Datenkommunikation zu verbinden.

Der zusätzliche Sensor kann beispielsweise zur Messung einer Atemanstrengung, eines Atemgeräuschs, einer Herzfrequenz, einer Blutsauerstoffsättigung, eines Blutdrucks, eines Blutzuckerspiegels, einer Körpertemperatur, eines Körpergewicht, einer Körperbewegung oder einer Kombination aus mindestens zwei dieser Beispiele konfiguriert sein.

Der zusätzliche Sensor kann konfiguriert sein, um seine Sensordaten direkt an die Datenkommunikationsschnittstelle des Beatmungsgeräts zu senden. Alternativ kann der zusätzliche Sensor konfiguriert sein, um seine Sensordaten über den Umweg eines mobilen Endgeräts wie beispielsweise eines Smartphones, einer Smartwatch oder eines Fitness- oder Schlaftrackers an die Datenkommunikationsschnittstelle zu senden. Dabei kann der zusätzliche Sensor in das mobile Endgerät integriert, d. h. in und/oder an dessen Gehäuse angeordnet sein.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Beatmungssystem gemäß einer Ausführungsform der Erfindung.
Fig. 2 zeigt Komponenten eines Sende- und Empfangssystems gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

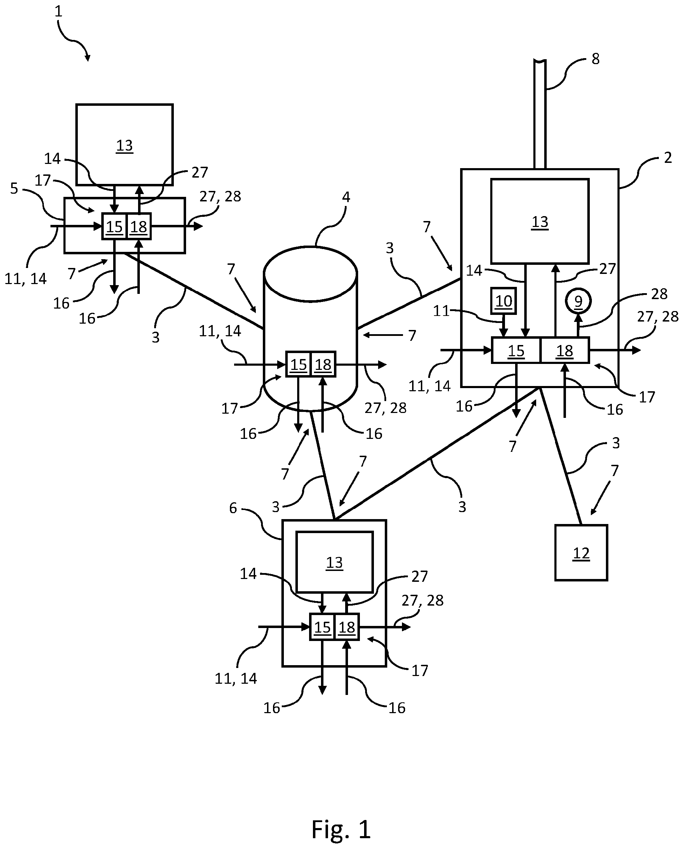

Fig. 1 zeigt ein Beatmungssystem 1, das ein Beatmungsgerät 2 über eine Datenkommunikationsverbindung 3 mit mehreren Datenverarbeitungseinrichtungen, hier einer ersten Datenverarbeitungseinrichtung 4 in Form eines (Cloud-)Servers 4, einer zweiten Datenverarbeitungseinrichtung 5 in Form eines PCs 5 und einer dritten Datenverarbeitungseinrichtung 6 in Form eines Smartphones 6, zur Datenkommunikation vernetzt, beispielsweise über das Internet, WLAN, Bluetooth und/oder Mobilfunk. Hierzu ist jedes der Geräte 2, 4, 5, 6 mit einer entsprechenden Datenkommunikationsschnittstelle 7 ausgestattet.

In diesem Beispiel verbindet die Datenkommunikationsverbindung 3 das Beatmungsgerät 2 jeweils über den Server 4 mit dem PC 5 und dem Smartphone 6. Möglich ist aber auch eine direkte Datenkommunikationsverbindung 3 zwischen dem Beatmungsgerät 2 und dem PC 5 und/oder, wie hier gezeigt, zwischen dem Beatmungsgerät 2 und dem Smartphone 6, beispielsweise über Bluetooth.

Das Beatmungsgerät 2 umfasst einen Beatmungsschlauch 8 zum Bereitstellen eines Atemgases, mit dem ein Patient invasiv oder nicht invasiv beatmet werden kann. Das Atemgas kann in seinem Druck und/oder Volumenstrom (Fluss) mittels eines geeigneten Aktors 9 des Beatmungsgeräts 2 steuerbar sein.

Zudem umfasst das Beatmungsgerät 2 einen Sensor 10 zum Erfassen von für die Beatmung, d. h. für die Steuerung des Atemdrucks und/oder -flusses, relevanten Parametern und zum Bereitstellen entsprechender Sensordaten 11. Die Sensordaten 11 können beispielsweise Messreihen für jeden Parameter, also dessen zeitlichen Verlauf, codieren.

Ein Teil der Sensordaten 11 kann optional von einem zusätzlichen, externen Sensor 12 bereitgestellt werden, der mit dem Beatmungsgerät 2 drahtgebunden und/oder drahtlos, vorzugsweise über Bluetooth und/oder WLAN, zur Datenkommunikation vernetzt sein kann. Der zusätzliche Sensor 12 kann konfiguriert sein, um über eine eigene Datenkommunikationsschnittstelle 7 unabhängig von anderen Netzwerkteilnehmern direkt mit dem Beatmungsgerät 2 zu kommunizieren. Möglich ist aber auch eine indirekte Datenkommunikation des zusätzlichen Sensors 12 mit dem Beatmungsgerät 2, hier beispielsweise über das Smartphone 6. Der zusätzliche Sensor 12 kann vom Patienten tragbar oder stationär sein.

Beispiele für die vorgenannten Parameter sind ein inspiratorischer und/oder exspiratorischer Druck, ein inspiratorischer und/oder exspiratorischer Volumenstrom, ein Atemgasvolumen pro Atemzug, eine Inspirations- und/oder Exspirationszeit, eine Beatmungsfrequenz, eine Atemfrequenz, eine Atemgaszusammensetzung, eine Atemanstrengung oder ein Atemgeräusch, eine Herzfrequenz, eine Blutsauerstoffsättigung, einen Blutdruck, einen Blutzuckerspiegel, eine Körpertemperatur, ein Körpergewicht oder eine Körperbewegung.

Beispielsweise kann der zusätzliche Sensor 12 konfiguriert sein, um Parameter wie die Atemfrequenz, die Atemanstrengung, das Atemgeräusch, die Herzfrequenz, die Blutsauerstoffsättigung, den Blutdruck, den Blutzuckerspiegel, die Körpertemperatur, das Körpergewicht oder die Körperbewegung zu erfassen.

Des Weiteren umfasst das Beatmungsgerät 2 eine Benutzerschnittstelle 13, hier in Form eines Touchdisplays, über das ein Benutzer (d. h. der Patient oder ein Bediener des Beatmungsgeräts 2) Informationen, insbesondere bezüglich der Beatmung (z. B. Bedienbefehle oder Angaben zur Benutzung), eingeben kann und das dem Benutzer Informationen, insbesondere bezüglich der Beatmung (z. B. gewählte Einstellungen oder Messwerte in Diagrammform), anzeigen kann.

Die Sensordaten 11 und/oder Benutzerdaten 14, die die über die Benutzerschnittstelle 13 eingegebenen Informationen codieren, werden als Eingabedaten in einem Sendemodul 15 des Beatmungsgeräts 2 empfangen, dort ausgewertet und je nach Auswertungsergebnis in eine Nachricht 16 umgewandelt, die dann über die Datenkommunikationsverbindung 3 an einen oder mehrere andere Netzwerkteilnehmer gesendet wird.

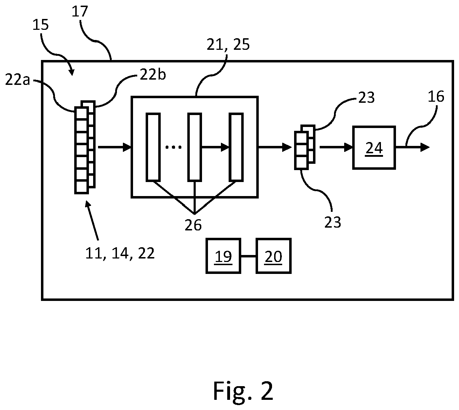

Das Sendemodul 15 (und alle nachstehend beschriebenen Module) kann als Hard- und/oder Software implementiert sein. In diesem Beispiel ist das Sendemodul 15 Teil eines Sende- und Empfangssystems 17 des Beatmungsgeräts 2, nachstehend kurz Steuerungssystem 17 genannt (siehe auch Fig. 2), das neben dem Sendemodul 15 ein Empfangsmodul 18 zum Empfangen einer Nachricht 16 umfasst.

Wie in Fig. 2 gezeigt, umfasst das Steuerungssystem 17 einen Speicher 19, in dem ein spezielles Computerprogramm gespeichert ist, und einen Prozessor 20, der konfiguriert ist, um Befehle des Computerprogramms auszuführen, wodurch das folgende Verfahren ausgeführt wird.

Nach dem Empfang der Eingabedaten im Sendemodul 15 werden die Eingabedaten ausgewertet, um ein bestimmtes Ereignis aus mehreren möglichen Ereignissen zu auszuwählen. Ein solches mögliches Ereignis kann beispielsweise eine medizinische und/oder technische Komplikation bei der Beatmung des Patienten sein.

Hierzu kann das Sendemodul 15 einen Klassifikator 21 ausführen, der aus den Eingabedaten (oder einem Teil davon) als Klassifikationsdaten 22 mehrere Klassenwerte 23 berechnet, wobei jeder Klassenwert 23 mit einer aus mehreren vordefinierten Ereignisklassen verknüpft ist und eine Wahrscheinlichkeit oder Tendenz für die jeweilige Ereignisklasse anzeigt. Die Klassenwerte 23 können dann in eine Auswerteeinheit 24 eingegeben werden, die anhand der Klassenwerte 23 eine der Ereignisklassen auswählt und die ausgewählte Ereignisklasse, genauer das der ausgewählten Ereignisklasse zugeordnete (mögliche) Ereignis, als erkanntes Ereignis bestimmt.

Hierauf generiert die Auswerteeinheit 24 eine Nachricht 16 bezüglich des erkannten Ereignisses und sendet diese über die Datenkommunikationsverbindung 3 an einen oder mehrere der anderen Netzwerkteilnehmer, hier an den Server 4 und/oder das Smartphone 6. Der PC 5 kann die Nachricht 16 dann vom Server 4 abrufen (dabei kann die Benutzerschnittstelle 13 des PCs 5 als Benutzerschnittstelle für den Server 4 fungieren).

Wie in Fig. 2 gezeigt, kann der Klassifikator 21 ein künstliches neuronales Netz 25 (nachstehend kurz Netz 25 genannt) mit mehreren trainierbaren Zwischenschichten 26 umfassen. Das Netz 25 kann mit beispielhaften Klassifikationsdaten, beispielsweise aufgezeichneten Sensor- und/oder Benutzerdaten, und entsprechenden Zielwerten trainiert worden sein, um die Klassifikationsdaten 22 in die Klassenwerte 23 umzuwandeln. Das Training kann online, d. h. im (betriebsfähigen) Beatmungsgerät 2, und/oder offline, d. h. außerhalb des Beatmungsgeräts 2, durchgeführt worden sein.

Vor der Eingabe ins Netz 25 können die Klassifikationsdaten 22 vorverarbeitet werden, um einen für die Verarbeitung durch das Netz 25 geeigneten Eingabevektor aus Eingabewerten zu erhalten. Zudem kann das Netz 25 konfiguriert sein, um die Klassenwerte 23 in Form eines für die weitere Verarbeitung in der Auswerteeinheit 24 geeigneten Ausgabevektors bereitzustellen.

In diesem Beispiel verarbeitet das Netz 25 zwei Sätze 22a, 22b der Klassifikationsdaten 22 (parallel und/oder sequenziell), wobei das Netz 25 für jeden der Sätze 22a, 22b einen Ausgabevektor mit Klassenwerten 23 erzeugt. Die Sätze 22a, 22b können sich beispielsweise in ihrem Datentyp voneinander unterscheiden. Beispielsweise kann ein erster Satz 22a einen anderen Typ Sensordaten 11 und/oder einen anderen Typ Benutzerdaten 14 umfassen als ein zweiter Satz 22b. Die Auswerteeinheit 24 wertet dann beide Ausgabevektoren aus, um das Ereignis zu erkennen und zu plausibilisieren.

Das Netz 25 kann auch mehr als zwei, beispielsweise mehr als zehn, mehr als hundert oder mehr als tausend Sätze der Klassifikationsdaten 22 (parallel und/oder sequenziell) verarbeiten, um das Ereignis zu erkennen und zu plausibilisieren.

Zusätzlich oder alternativ kann die Erkennung des Ereignisses basierend auf einem oder mehreren Schwellenwertvergleichen erfolgen.

Es ist möglich, dass unterschiedliche erkannte Ereignisse unterschiedlich wichtig für die Beatmung des Patienten sind. Dementsprechend kann eine Nachricht 16, die sich auf ein wichtiges Ereignis bezieht, mit höherer Priorität gesendet werden als eine Nachricht 16, die sich auf ein weniger wichtiges Ereignis bezieht.

Hierzu kann beispielsweise jeder der vorgenannten Ereignisklassen eine bestimmte Sendepriorität zugeordnet sein, die beim Senden der jeweiligen Nachricht 16 berücksichtigt wird.

Zusätzlich oder alternativ können für jede Nachricht 16 bestimmte Datenparameter, die die beim Senden der Nachricht 16 anfallende Datenmenge beeinflussen, individuell in Abhängigkeit vom Inhalt der Nachricht 16, d. h. vom erkannten Ereignis, eingestellt werden.

Die Nachricht 16 kann dann von einem Empfangsmodul 18 in einer oder mehreren der Datenverarbeitungseinrichtungen 4, 5, 6 empfangen, dort ausgewertet und in einen oder mehrere Steuerbefehle umgesetzt werden, genauer in einen ersten Steuerbefehl 27 zum Ansteuern einer Benutzerschnittstelle 13 der jeweiligen Datenverarbeitungseinrichtung und/oder in einen zweiten Steuerbefehl 28 zum Ansteuern des Aktors 9. Hierzu kann das Empfangsmodul 18 einen der oder beide Steuerbefehle 27, 28 über die Datenkommunikationsverbindung 3 ans Beatmungsgerät 2 und/oder an mindestens einen der anderen Netzwerkteilnehmer senden. Der jeweilige Netzwerkteilnehmer kann dann beispielsweise seine Benutzerschnittstelle 13 entsprechend dem ersten Steuerbefehl 27 so ansteuern, dass dem jeweiligen Benutzer bestimmte Inhalte aus der Nachricht 16 wiedergegeben werden.

Es ist möglich, dass mindestens eine oder, wie hier, jede der Datenverarbeitungseinrichtungen 4, 5, 6 mit einem Sende- und Empfangssystem 17 ausgestattet ist, d. h. Nachrichten 16 empfangen und senden kann. In diesem Fall kann das Empfangsmodul 18 des Beatmungsgeräts 2 konfiguriert sein, um die Nachrichten 16 von der jeweiligen Datenverarbeitungseinrichtung zu empfangen und wie vorstehend beschrieben in erste Steuerbefehle 25 und/oder zweite Steuerbefehle 26 umzusetzen (und diese gegebenenfalls über die Datenkommunikationsverbindung 3 weiterzuleiten).

Möglich ist in diesem Fall auch, dass mindestens einer oder, wie hier, jeder der Netzwerkteilnehmer zumindest einen Teil der Benutzerdaten 14, die eine Benutzerschnittstelle 13 mindestens eines anderen Netzteilnehmers ausgegeben hat, und/oder zumindest einen Teil der Sensordaten 11, die mindestens einer der Sensoren 10, 12 ausgegeben hat, über die Datenkommunikationsverbindung 3 empfängt und als die Eingabedaten verwendet.

Ereignisse, die das Sendemodul 15 im Fall einer nicht optimal eingestellten Beatmung und/oder einer Änderung des Beatmungsbedarfs erkennen kann, können beispielsweise sein:
- das Beatmungsgerät 2 und der Patient sind nicht synchron;
- die Druckunterstützung durch das Beatmungsgerät 2 ist zu groß oder zu klein;
- der vom Beatmungsgerät 2 erzeugte Schienungsdruck (d. h. der exspiratorische Druck) ist zu klein;
- das Beatmungsgerät 2 behindert die Ausatmung des Patienten;
- die Druckrampen zwischen exspiratorischem und inspiratorischem Druck zu Beginn oder am Ende der Inspiration sind unphysiologisch, d. h., der Druck ändert sich zu schnell oder zu langsam;
- der Patient hat Atemnot bei der Beatmung oder nach dem Abschalten der Beatmungsfunktion;
- der Beatmungsbedarf des Patienten hat sich deutlich geändert (z. B. aufgrund körperlicher oder sportlicher Aktivität, aufgrund eines Infekts, beim Aufstehen, beim Schlafengehen).

Ereignisse, die das Sendemodul 15 bei der Applikation der Beatmung auf die Atemwege erkennen kann, können beispielsweise sein:
- das Patienteninterface ist undicht und/oder der Patient atmet mit offenem Mund, sodass kein ausreichender Druck auf die unteren Atemwege appliziert werden kann;
- Austrocknung der Atemwege;
- unzureichende Motivation des Patienten;
- Probleme bei der Bedienung des Beatmungsgeräts 2;
- das Beatmungsgerät 2 stört mit seiner Ausrüstung den Schlaf des Patienten;
- der Patient hat Kopfschmerzen;
- der Patient hat Sinusitis.

Diese Ereignisse können insbesondere erkannt werden durch:
- Auswerten der Atemflusskurve allein oder in Kombination mit der Atemdruckkurve (hierzu können beispielsweise Sensordaten 11 mit besonders hoher Auflösung in einer Nachricht 16 versendet werden);
- Auswerten der Atemanstrengung, die aus der Atemflusskurve und der Atemdruckkurve berechnet und/oder mittels eines Effort-Sensors gemessen werden kann;
- Auswerten von durch den Patienten über eine Benutzerschnittstelle 13 eingegebenen Informationen;
- die Applikation eines oder mehrerer zusätzlicher Sensoren 12 und Auswerten der jeweiligen Sensordaten 11 infolge eines Anfangsverdachts, der sich beim erstmaligen Auswerten der Eingabedaten ergab.

Die Nachricht 16 kann in solchen Fällen beispielsweise eine oder mehrere der folgenden Informationen umfassen:
- hochaufgelöste Abschnitte der Sensordaten 11, beispielsweise zur visuellen Auswertung durch einen Experten, der dann über eine geeignete Intervention entscheiden kann;
- einen Vorschlag für eine Eigenintervention, die der Patient selbst durchführen kann;
- Einstellungen zur automatischen Optimierung der Beatmungsfunktion;
- eine Anleitung zur Optimierung oder zum Tausch von Beatmungszubehör wie z. B. dem Beatmungsschlauch 8 oder einer Beatmungsmaske;
- eine motivierende Botschaft;
- eine Aufforderung an den Patienten, Kontakt mit einer Kontaktperson aufzunehmen;
- eine Einladung zu einer Beratung des Patienten über eine spezielle App per Chat und/oder Videotelefonie.

Darüber hinaus kann die Nachricht 16 bei einer erkannten akuten Verschlechterung einer bestehenden Erkrankung des Patienten, beispielsweise von Herz, Lunge oder Hirnstamm, speziell an einen medizinischen Betreuer gerichtete Informationen für weitere Interventionen zusätzlich zur Beatmung umfassen.

Nachstehend werden verschiedene Ausführungsformen der Erfindung nochmals mit anderen Worten beschrieben.

Das Beatmungsgerät 2 kann konfiguriert sein, um durch mindestens einen Sensor 10, 12 erfasste Sensordaten 11, insbesondere Flow- und Druckkurven, oder daraus abgeleitete Signale zu ermitteln und zu speichern. Solche Signale können beispielsweise mindestens eine der folgenden Informationen anzeigen: Leckage, Atemfrequenz, Tidalvolumen, Atemereignisse, Synchronitätsindikatoren, Schlafstadienindikatoren, Hustenindikatoren, Sekretbildungsindikatoren, Umgebungsbedingungen (z. B. Temperatur, Luftfeuchte, Luftdruck).

Zusätzlich oder alternativ können Daten optionaler externer Sensoren ermittelt bzw. gespeichert werden. Diese Daten können beispielsweise mindestens eine der folgenden Informationen anzeigen: Kohlenstoffdioxidgehalt der Ausatemluft, des Bluts oder der Umgebung, Sauerstoffgehalt der Einatemluft, des Bluts oder der Umgebung, Körpertemperatur, EKG-Signale, Bewegungssignale (z. B. erzeugt durch Beschleunigungs-, Radar-, Ultraschall-, Abstands- oder Drucksensoren, beispielsweise im Bett, oder durch elektromagnetische Felder), Informationen über Abläufe wie Therapiestart oder -ende, Konfigurationsdaten, Sendevorgänge, technische Fehler, Ergebnisse von Selbsttests, Abnutzungsdaten, Füllstände eines Wasser- oder Sauerstoffreservoirs.

Diese Sensordaten werden als Eingabedaten definiert und ausgewertet, um ein Ereignis unter mehreren möglichen Ereignissen zu erkennen, wobei jedes mögliche Ereignis für eine Beatmung des Patienten durch das Beatmungsgerät 2 relevant ist.

Wird ein Ereignis erkannt, so wird das Beatmungsgerät dazu veranlasst, Sensordaten, die in einem bestimmten Zeitraum gespeichert wurden, aus dem Speicher auszulesen und mit einer bestimmten, beispielsweise abhängig vom jeweiligen Ereignistyp definierten Auflösung über eine Datenkommunikationsverbindung 3 an ein Empfangsmodul 18 zu senden, wobei die Datenkommunikationsverbindung 3 das Beatmungsgerät 2 mit mindestens einer außerhalb des Beatmungsgeräts 2 befindlichen Datenverarbeitungseinrichtung 4, 5, 6 zur Datenkommunikation verbindet.

Der Zeitraum kann pro Ereignis vordefiniert sein. Beispielsweise kann der Zeitraum einen festen ersten Zeitraum vor und/oder einen festen zweiten Zeitraum nach dem Zeitpunkt des Ereignisses umfassen (bei einem Hustenstoß z. B. zwei Minuten davor und/oder danach). Der Zeitraum kann auch abhängig vom letzten Sendevorgang definiert sein. Alternativ können die Daten seit dem letzten Sendevorgang versandt werden, sobald das Ereignis eintritt.

Da der Zeitraum zum Zeitpunkt der Erkennung des Ereignisses möglicherweise noch nicht abgeschlossen ist, kann der Sendevorgang in manchen Fällen verzögert ausgelöst werden. Wenn beispielsweise bei einem Hustenstoß als erkanntem Ereignis Daten bezüglich des Ereignisses selbst und bezüglich eines bestimmten Zeitraums vor und nach dem Ereignis (z. B. jeweils 2 Minuten) hochaufgelöst übertragen werden sollen, kann zum Zeitpunkt der Erkennung des Ereignisses ein Timer über 2 Minuten gestartet werden, nach dessen Ablauf schließlich 4 Minuten hochaufgelöste Daten übertragen werden.

Alternativ zum sofortigen Versenden nach Ablauf des Zeitraums können die Daten zunächst in einem Sendepuffer zwischengespeichert, d. h. für ein Versenden vorgemerkt werden. Wenn dann das nächste Sendeereignis eintritt, beispielsweise turnusmäßig mehrere Male pro Tag nach einer Aufforderung zum Senden durch den Benutzer oder die Datenverarbeitungseinrichtung, werden alle Einträge des Sendepuffers übertragen. Beispielsweise werden immer dann, wenn das Beatmungsgerät einen Atemaussetzer erkennt, 30, 60, 90 oder 180 (oder mehr) Sekunden hochaufgelöste Daten vor und nach diesem Ereignis im Sendepuffer zwischengespeichert. Bei der nächsten Übertragung werden die Einträge gemeinsam gesendet. Alternativ wird pro Ereignistyp eine vorher festgelegte Anzahl von Beispielen, beispielsweise die ersten 5, 10, 20 oder 100 Beispiele, als Repräsentanten im Sendepuffer zwischengespeichert, damit die Datenverarbeitungseinrichtung und/oder ein menschlicher Auswerter anhand der Beispiele analysieren kann, was genau bei der Beatmung passiert.

Optional kann der Langzeitspeicher für die (hochaufgelösten) Daten im Gerät rollierend gelöscht werden, beispielsweise nach einer bestimmten Anzahl von Stunden oder Tagen. Dabei kann die Speicherdauer gleich der Maximallänge des zu versendenden Zeitraums über alle definierten Ereignistypen sein.

Es ist möglich, dass der Sendevorgang bei bestimmten Ereignissen nicht schon beim ersten Auftreten, sondern erst nach mehrmaligem Auftreten, d. h., wenn eine bestimmte Häufigkeit erreicht wird (z. B. 2-mal pro Minute, 10-mal pro Stunde oder 5-mal pro Tag), ausgelöst wird.

Beispiele für technische Ereignisse: Ablauf des Timers für die Datenübertragung; Anfrage von Daten durch das Empfangsmodul einer Gegenstelle; technischer Alarm oder Fehler; Erreichen einer bestimmten Anzahl von Alarmen/Fehlern; Sendefehler bezüglich eines bestimmten Sendewegs (somit Triggern eines anderen Sendewegs); Therapiestart oder -stopp; Veränderung der Therapieeinstellungen; zu lange oder zu kurze Therapiepause; unzulässige Umgebungsbedingungen; Blockade von Beatmungsschlauch, Patienteninterface und/oder Geräteeinlass; zu hohe Leckage.

Manuelle Ereignisse können vom Benutzer über die Benutzerschnittstelle eingegeben werden, beispielsweise durch den Patienten selbst oder eine sonstige Person, die sich in der Nähe des Patienten oder auch nicht in der Nähe des Patienten befindet, wie einen Betreuer, einen Experten oder einen Angehörigen. Die Auslösung kann über die Benutzerschnittstelle beispielsweise direkt am Gerät und/oder über eine Fernsteuerung, einen Netzwerkbefehl, eine App, eine Cloudanwendung oder eine SMS erfolgen.

Beispiele für medizinische Ereignisse: Atemaussetzer, Hustenstöße, Veränderungen des Kohlenstoffdioxid- oder Sauerstoffgehalts, Arrhythmie des Herzens, Aussetzen des Herzschlags, Diskonnektion der Beatmung, Diskonnektion eines zusätzlichen Sensors, Veränderung der Körpertemperatur, Meldung einer Komplikation oder einer Verschlimmerung eines Symptoms des Patienten über eine Mensch-Maschine-Schnittstelle am Beatmungsgerät oder an einem anderen Modul (z. B. einem mobilen Endgerät), erhöhte Sekretmenge in den Atemwegen, Unterschreiten eines Atemzug- oder Atemzeitvolumens.

Die Ereignisse können einzeln oder in Kombination erkannt werden.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder

Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Beatmungssystem
- 2: Beatmungsgerät
- 3: Datenkommunikationsverbindung
- 4: erste Datenverarbeitungseinrichtung / Server
- 5: zweite Datenverarbeitungseinrichtung / PC
- 6: dritte Datenverarbeitungseinrichtung / Smartphone
- 7: Datenkommunikationsschnittstelle
- 8: Beatmungsschlauch
- 9: Aktor
- 10: Sensor
- 11: Sensordaten
- 12: zusätzlicher Sensor
- 13: Benutzerschnittstelle
- 14: Benutzerdaten
- 15: Sendemodul
- 16: Nachricht
- 17: Sende- und Empfangssystem / Steuerungssystem
- 18: Empfangsmodul
- 19: Speicher
- 20: Prozessor
- 21: Klassifikator
- 22: Klassifikationsdaten
- 22a: erster Satz
- 22b: zweiter Satz
- 23: Klassenwert
- 24: Auswerteeinheit
- 25: künstliches neuronales Netz
- 26: Zwischenschicht
- 27: erster Steuerbefehl
- 28: zweiter Steuerbefehl

## Patentansprüche

1. Sendemodul (15), umfassend Mittel (19, 20) zum:
Empfangen von Eingabedaten (11, 14), umfassend: Sensordaten (11), die mindestens einen durch mindestens einen Sensor (10, 12) erfassten und für eine Beatmung eines Patienten durch ein Beatmungsgerät (2) relevanten Parameter definieren, und/oder Benutzerdaten (14), die durch einen Benutzer über eine Benutzerschnittstelle (13) eingegebene Informationen bezüglich eines durch ein Beatmungsgerät (2) beatmeten oder zu beatmenden Patienten codieren;
Auswerten der Eingabedaten (11, 14), um ein Ereignis unter mehreren möglichen Ereignissen zu erkennen, wobei jedes mögliche Ereignis für eine Beatmung des Patienten durch das Beatmungsgerät (2) relevant ist;
Senden einer Nachricht (16) bezüglich des erkannten Ereignisses an ein Empfangsmodul (18) über eine Datenkommunikationsverbindung (3), die das Beatmungsgerät (2) mit mindestens einer außerhalb des Beatmungsgeräts (2) befindlichen Datenverarbeitungseinrichtung (4, 5, 6) zur Datenkommunikation verbindet.

2. Sendemodul (15) nach Anspruch 1,
wobei ein erster Satz (22a) der Eingabedaten (11, 14) empfangen wird und die Eingabedaten (11, 14) aus dem ersten Satz (22a) ausgewertet werden, um das Ereignis zu erkennen;
wobei das Auswerten der Eingabedaten (11, 14) zusätzlich umfasst, wenn das Ereignis erkannt wird:
Anfordern mindestens eines vom ersten Satz (22a) abweichenden zweiten Satzes (22b) der Eingabedaten (11, 14);
wenn der zweite Satz (22b) empfangen wird: Prüfen anhand der Eingabedaten (11, 14) aus dem zweiten Satz (22b), ob das erkannte Ereignis plausibel ist, wobei ein Prüfergebnis erzeugt wird;
wobei die Nachricht (16) abhängig vom Prüfergebnis gesendet wird.

3. Sendemodul (15) nach einem der vorhergehenden Ansprüche,
wobei das Auswerten der Eingabedaten (11, 14) umfasst:
Umwandeln zumindest eines Teils der Eingabedaten (11, 14) als Klassifikationsdaten (22) in Klassenwerte (23) durch einen Klassifikator (21), wobei jeder Klassenwert (23) eine Wahrscheinlichkeit und/oder Tendenz für eine von mehreren Ereignisklassen anzeigt, wobei jede Ereignisklasse einem der möglichen Ereignisse entspricht;
Erkennen des Ereignisses unter Verwendung der Klassenwerte (23).

4. Sendemodul (15) nach Anspruch 3,
wobei der Klassifikator (21) ein künstliches neuronales Netz (25) umfasst, das mit beispielhaften Klassifikationsdaten trainiert wurde, um die Klassifikationsdaten (22) in die Klassenwerte (23) umzuwandeln.

5. Sendemodul (15) nach einem der vorhergehenden Ansprüche,
wobei eine Abweichung des mindestens einen Parameters von mindestens einem Sollwert bestimmt wird und das Ereignis abhängig von der Abweichung erkannt wird.

6. Sendemodul (15) nach einem der vorhergehenden Ansprüche,
wobei jedem möglichen Ereignis eine Priorität zugeordnet ist und die Nachricht (16) entsprechend der Priorität des erkannten Ereignisses gesendet wird.

7. Sendemodul (15) nach einem der vorhergehenden Ansprüche,
wobei ein Wert für mindestens einen Datenparameter abhängig vom erkannten Ereignis bestimmt wird, wobei der mindestens eine Datenparameter die Menge an Daten, die beim Senden der Nachricht (16) gesendet werden, definiert.

8. Sendemodul (15) nach einem der vorhergehenden Ansprüche,
wobei die möglichen Ereignisse mindestens eines der folgenden Ereignisse umfassen:
eine Abweichung einer Beatmungsfrequenz, mit der der Patient beatmet wird, von einer Atemfrequenz, mit der der Patient atmet, liegt außerhalb eines Sollbereichs;
ein inspiratorischer und/oder exspiratorischer Druck liegt außerhalb eines Sollbereichs;
eine Geschwindigkeit, mit der sich ein Druck beim Übergang zwischen Inspiration und Exspiration ändert, liegt außerhalb eines Sollbereichs;
ein Druckverlust bei der Beatmung liegt außerhalb eines Sollbereichs;
der Patient hat Atemnot bei der Beatmung und/oder als Reaktion auf die Beendigung der Beatmung;
das Beatmungsgerät (2) erschwert das Aus- und/oder Einatmen;
der Patient hat Schlafprobleme;
der Patient hat Kopfschmerzen;
der Patient hat zu trockene Atemwege;
der Patient ist erkältet;
eine bestehende Erkrankung des Patienten hat sich signifikant verschlechtert oder verbessert;
der Patient hat Schwierigkeiten bei der Bedienung des Beatmungsgeräts (2);
die Compliance des Patienten ist unzureichend.

9. Sendemodul (15) nach einem der vorhergehenden Ansprüche,
wobei die Nachricht (16) mindestens eine der folgenden Informationen umfasst:
zumindest einen Teil der ausgewerteten Eingabedaten (11, 14);
eine Beschreibung des erkannten Ereignisses;
eine Aufforderung an einen Benutzer, Informationen bezüglich des erkannten Ereignisses über eine Benutzerschnittstelle (13) einzugeben;
eine Aufforderung an den Patienten, Kontakt mit einer Kontaktperson aufzunehmen, insbesondere über einen Chat- und/oder Videokommunikationsdienst;
eine Anleitung, wie eine bestimmte Komplikation bei der Beatmung beseitigt und/oder verhindert werden kann;
eine Ermunterung, die den Patienten zu einer besseren Compliance motivieren soll;
einen an das erkannte Ereignis angepassten Wert für mindestens einen Steuerparameter zum Steuern eines Aktors (9) des Beatmungsgeräts (2);
eine Patientenkennung zur eindeutigen Identifizierung des Patienten;
eine Gerätekennung zur eindeutigen Identifizierung des Beatmungsgeräts (2).

10. Empfangsmodul (18), umfassend Mittel (19, 20) zum:
Empfangen einer Nachricht (16) bezüglich eines für eine Beatmung eines Patienten durch ein Beatmungsgerät (2) relevanten Ereignisses von einem Sendemodul (15) über eine Datenkommunikationsverbindung (3), die das Beatmungsgerät (2) mit mindestens einer außerhalb des Beatmungsgeräts (2) befindlichen Datenverarbeitungseinrichtung (4, 5, 6) zur Datenkommunikation verbindet;
Erzeugen eines ersten Steuerbefehls (27) zum Ansteuern einer Benutzerschnittstelle (13), sodass eine für einen Benutzer relevante Information aus der Nachricht (16) über die Benutzerschnittstelle (13) wiedergegeben wird; und/oder
Erzeugen eines zweiten Steuerbefehls (28) zum Ansteuern eines Aktors (9) des Beatmungsgeräts (2) unter Verwendung der Nachricht (16), wobei der Aktor (9) ausgebildet ist, um einen Atemgasdruck und/oder einen Atemgasvolumenstrom zum Beatmen des Patienten bereitzustellen.

11. Sende- und Empfangssystem (17), umfassend:
ein Sendemodul (15) nach einem der Ansprüche 1 bis 9;
ein Empfangsmodul (18) nach Anspruch 10.

12. Beatmungsgerät (2), umfassend:
einen Sensor (10) zum Erfassen von mindestens einem für eine Beatmung eines Patienten durch das Beatmungsgerät (2) relevanten Parameter;
eine Datenkommunikationsschnittstelle (7) zum Verbinden des Beatmungsgeräts (2) mit mindestens einer außerhalb des Beatmungsgeräts (2) befindlichen Datenverarbeitungseinrichtung (4, 5, 6) zur Datenkommunikation;
eine Benutzerschnittstelle (13) zum Eingeben von Informationen durch einen Benutzer und/oder zum Wiedergeben von für einen Benutzer relevanten Informationen;
einen Aktor (9) zum Bereitstellen eines Atemgasdrucks und/oder eines Atemgasvolumenstroms zum Beatmen des Patienten;
ein Sendemodul (15) nach einem der Ansprüche 1 bis 9 und/oder ein Empfangsmodul (18) nach Anspruch 10.

13. Beatmungsgerät (2) nach Anspruch 12,
wobei die Datenkommunikationsschnittstelle (7) konfiguriert ist, um das Beatmungsgerät (2) zusätzlich mit mindestens einem außerhalb des Beatmungsgeräts (2) befindlichen und/oder vom Patienten getragenen zusätzlichen Sensor (12) zum Erfassen von mindestens einem für die Beatmung des Patienten relevanten Parameter zur Datenkommunikation zu verbinden.

14. Beatmungssystem (1), umfassend:
ein Beatmungsgerät (2) nach Anspruch 12 oder 13;
eine außerhalb des Beatmungsgeräts (2) befindliche Datenverarbeitungseinrichtung (4, 5, 6), umfassend:
eine Datenkommunikationsschnittstelle (7) zum Verbinden der Datenverarbeitungseinrichtung (4, 5, 6) mit dem Beatmungsgerät (2) zur Datenkommunikation,
eine Benutzerschnittstelle (13) zum Eingeben von Informationen durch einen Benutzer und/oder zum Wiedergeben von für einen Benutzer relevanten Informationen,
ein Sendemodul (15) nach einem der Ansprüche 1 bis 9 und/oder ein Empfangsmodul (18) nach Anspruch 10.

15. Computerprogramm, umfassend einen ersten Satz von Befehlen, die ein Sendemodul (15) bei der Ausführung des Computerprogramms durch das Sendemodul (15) veranlassen zum:
Empfangen von Eingabedaten (11, 14), umfassend: Sensordaten (11), die mindestens einen durch mindestens einen Sensor (10, 12) erfassten und für eine Beatmung eines Patienten durch ein Beatmungsgerät (2) relevanten Parameter definieren, und/oder Benutzerdaten (14), die durch einen Benutzer über eine Benutzerschnittstelle (13) eingegebene Informationen bezüglich eines durch ein Beatmungsgerät (2) beatmeten oder zu beatmenden Patienten codieren;
Auswerten der Eingabedaten (11, 14), um ein Ereignis unter mehreren möglichen Ereignissen zu erkennen, wobei jedes mögliche Ereignis für eine Beatmung des Patienten durch das Beatmungsgerät (2) relevant ist;
Senden einer Nachricht (16) bezüglich des erkannten Ereignisses an ein Empfangsmodul (18) über eine Datenkommunikationsverbindung (3), die das Beatmungsgerät (2) mit mindestens einer außerhalb des Beatmungsgeräts (2) befindlichen Datenverarbeitungseinrichtung (4, 5, 6) zur Datenkommunikation verbindet;
und/oder umfassend einen zweiten Satz von Befehlen, die ein Empfangsmodul (18) bei der Ausführung des Computerprogramms durch das Empfangsmodul (18) veranlassen zum:
Empfangen einer Nachricht (16) bezüglich eines für eine Beatmung eines Patienten durch ein Beatmungsgerät (2) relevanten Ereignisses von einem Sendemodul (15) über eine Datenkommunikationsverbindung (3), die das Beatmungsgerät (2) mit mindestens einer außerhalb des Beatmungsgeräts (2) befindlichen Datenverarbeitungseinrichtung (4, 5, 6) zur Datenkommunikation verbindet;
Erzeugen eines ersten Steuerbefehls (27) zum Ansteuern einer Benutzerschnittstelle (13), sodass eine für einen Benutzer relevante Information aus der Nachricht (16) über die Benutzerschnittstelle (13) wiedergegeben wird; und/oder
Erzeugen eines zweiten Steuerbefehls (28) zum Ansteuern eines Aktors (9) des Beatmungsgeräts (2) unter Verwendung der Nachricht (16), wobei der Aktor (9) ausgebildet ist, um einen Atemgasdruck und/oder einen Atemgasvolumenstrom zum Beatmen des Patienten bereitzustellen.

16. Computerlesbares Medium, auf dem das Computerprogramm nach Anspruch 15 gespeichert ist.

17. Verfahren zum Betreiben eines Sendemoduls (15) und/oder eines Empfangsmoduls (18), wobei das Verfahren zum Betreiben des Sendemoduls (15) umfasst:
Empfangen von Eingabedaten (11, 14), umfassend: Sensordaten (11), die mindestens einen durch mindestens einen Sensor (10, 12) erfassten und für eine Beatmung eines Patienten durch ein Beatmungsgerät (2) relevanten Parameter definieren, und/oder Benutzerdaten (14), die durch einen Benutzer über eine Benutzerschnittstelle (13) eingegebene Informationen bezüglich eines durch ein Beatmungsgerät (2) beatmeten oder zu beatmenden Patienten codieren;
Auswerten der Eingabedaten (11, 14), um ein Ereignis unter mehreren möglichen Ereignissen zu erkennen, wobei jedes mögliche Ereignis für eine Beatmung des Patienten durch das Beatmungsgerät (2) relevant ist;
Senden einer Nachricht (16) bezüglich des erkannten Ereignisses an ein Empfangsmodul (18) über eine Datenkommunikationsverbindung (3), die das Beatmungsgerät (2) mit mindestens einer außerhalb des Beatmungsgeräts (2) befindlichen Datenverarbeitungseinrichtung (4, 5, 6) zur Datenkommunikation verbindet;
und/oder wobei das Verfahren zum Betreiben des Empfangsmoduls (18) umfasst:
Empfangen einer Nachricht (16) bezüglich eines für eine Beatmung eines Patienten durch ein Beatmungsgerät (2) relevanten Ereignisses von einem Sendemodul (15) über eine Datenkommunikationsverbindung (3), die das Beatmungsgerät (2) mit mindestens einer außerhalb des Beatmungsgeräts (2) befindlichen Datenverarbeitungseinrichtung (4, 5, 6) zur Datenkommunikation verbindet;
Erzeugen eines ersten Steuerbefehls (27) zum Ansteuern einer Benutzerschnittstelle (13), sodass eine für einen Benutzer relevante Information aus der Nachricht (16) über die Benutzerschnittstelle (13) wiedergegeben wird; und/oder
Erzeugen eines zweiten Steuerbefehls (28) zum Ansteuern eines Aktors (9) des Beatmungsgeräts (2) unter Verwendung der Nachricht (16), wobei der Aktor (9) ausgebildet ist, um einen Atemgasdruck und/oder einen Atemgasvolumenstrom zum Beatmen des Patienten bereitzustellen.
